# EUROPEAN PATENT SPECIFICATION

(11) **EP 4 498 810 B1**
(45) Date of publication and mention of the grant of the patent: **21.10.2026**
(21) Application number: 24734786.7
(22) Date of filing: 28.03.2024
(51) Int. Cl.: A01N 1/147, C12N 5/00, G06K 19/07

(54) **CRYOPRESERVATION DEVICE WITH INTEGRATED TRACKING DEVICE CHAMBER**
KRYOKONSERVIERUNGSVORRICHTUNG MIT INTEGRIERTER VERFOLGUNGSVORRICHTUNGSKAMMER
DISPOSITIF DE CRYOCONSERVATION À CHAMBRE DE DISPOSITIF DE SUIVI INTÉGRÉE

(30) Priority: 19.04.2023 US 202318136419
(43) Date of publication of application: 05.02.2025
(60) Divisional application: 26159921.1 / 4 726 602
(73) Proprietor: Biotech, Inc., Alpharetta, GA 30005 (US)
(72) Inventor: PARRA-ALMANZA, Jorge, E., Alpharetta, GA 30005 (US)
(74) Representative: dompatent
(86) International application number: PCT/US2024/021859
(87) International publication number: WO 2024/220212

(56) References cited:
- CN-A- 108 541 702
- CN-U- 215 867 929
- US-A1- 2022 184 625
- US-A1- 2022 361 483
- US-A1- 2022 388 005

## Description

### CROSS-REFERENCE

This application claims the benefit of U.S. Non-Provisional Application No. 18/136,419, filed on April 19, 2023.

### FIELD OF THE INVENTION

The present invention relates generally to the field of devices for the cryopreservation of biological specimens. More specifically, it relates to devices capable of being remotely identified through RFID technology.

### BACKGROUND OF THE INVENTION

Cryopreservation is practiced in the life sciences for the purpose of halting biological activity in viable cells for an extended period of time. Among the techniques used for cryopreservation is vitrification.

Vitrification involves the transformation of a solution comprised of a biological specimen, i.e., an oocyte or an embryo, into a glass-like amorphous solid that is free from any crystalline structure, followed by extremely rapid cooling. One of the major challenges of this method is to prevent the intracellular liquid within the oocyte or embryo to form ice crystals. Accordingly, the first step is to dehydrate the cell or cells as much as possible using cryoprotectant containing fluids called "vitrification media." The biological specimen is then rapidly chilled by immersion in a cryogenic fluid such as liquid nitrogen (LN₂). With a proper combination of chilling speed and cryoprotectant concentration, intracellular water will attain a solid, innocuous, glassy (vitreous) state rather than an orderly, damaging, crystalline ice state. Vitrification can be described as a rapid increase in fluid viscosity that traps the water molecules in a random orientation. Vitrification media, however, can contain relatively high levels of cryoprotectant that can be toxic to cells except in the vitreous state. As a result, the time exposure of cells to vitrification media during dehydration and warming must be carefully controlled to avoid cellular injury, and, accordingly, it is desirable to chill the specimen as quickly as possible.

The CRYOLOCK cryopreservation device was developed as a versatile, simple, and efficient vitrification device that is intended for holding, cryopreservation, and storage of oocytes or embryos in liquid nitrogen. It is a cryopreservation device which allows the specimen to be rapidly cooled and stored without direct contact with the aseptic liquid nitrogen (LN₂). The CRYOLOCK cryopreservation device is described in U.S. Patent Application Publication number US 2016/0174545, published on June 23, 2016.

The CRYOLOCK cryopreservation device uses the combination of semi-flexible plastic and a functional design, including specific shapes and close clearances, to hermetically seal a specimen within the cryopreservation device at room temperature and maintain the hermetic seal as the cryopreservation device and specimen cool to the temperature of the liquid nitrogen.

Due to the expense of storing specimens in liquid nitrogen, multiple samples are stored in a given cryostorage tank. As entire cryopreservation devices are stored in the cryostorage tank, the size of each cryopreservation device and the packing characteristics of such cryopreservation devices impact the amount to space required, and thus, the cost of storage. On top of the simple space concern, there is a need for ongoing quality management and audits to ensure tracking of the identity and storage conditions of each sample over time. If cryopreservation devices need to be removed from the cryostorage device during a quality audit, the temperature swings can impact the long-term viability of the specimen.

CN 108541702 A discloses a cryopreservation rod with a coded information chip, which is composed of a rod cap (1) and a rod body (2). The rod body (2) is composed of an information recording end (2-3) and a grip handle (2-2). The left end of the rod body (2) is formed with a carrier concave sheet (2-1), wherein the carrier concave sheet (2-1) is made of a polymer sheet. The information recording end (2-3) is formed on the right end, and the connection between the information recording end (2-3) and the rod body (2) is connected by a tapered surface; the information recording end (2-3) comprises an empty tube (2-3-1), an RFID coded information chip (2-3-2) and an empty tube plug (2-3-3). The open end is sealed by thermoplastic bonding.

There is a need to be able to identify each sample/device in a cryostorage tank without removing the cryopreservation device from the cryostorage tank. There is a need to maintain or reduce the size of cryopreservation devices that are able to self-identify, particularly at the single biological specimen level. There is a need to maintain the ability to identify each specimen/device over the life cycle of the gathering, storage, retrieval, and use of each specimen.

Incorporating an RFID tag in each cryopreservation device presents several challenges. First, the part of the device that holds the biological specimen must be sterilized, and it typically is sterilized with gamma radiation which can damage the RFID tag's ability to function. Also, the incorporation of the RFID tag must fit into the flow of work during vitrification process, which means the RFID tag needs to be securely engaged with a part of the cryopreservation device before the biological specimen is collected as the time between collecting the specimen and immersion in liquid nitrogen is necessarily short. The RFID tag needs to be securely held by the cryopreservation device at ambient conditions and at the temperature of liquid nitrogen, without causing damage to the cryopreservation device upon such large temperature swings. There is a need to minimize any size increase in the cryopreservation device capable of holding an RFID tag. Ideally, the RFID tag can be secured to the cryopreservation device without the need for an adhesive which may be impacted by the low temperatures.

### BRIEF SUMMARY OF THE INVENTION

According to the present invention, a cryopreservation device capable of holding a Radio Frequency Identification (RFID) tag as defined in claim 1 is provided. Particular embodiments are defined in the dependent claims. The cryopreservation device comprises a) an elongated stick comprising an RFID section, an elongated body, and a specimen collection tip, and b) a cap comprising a hollow chamber, wherein the hollow chamber has a length sufficient to accommodate the specimen collection tip. The cap is capable of enclosing the specimen collection tip within the hollow chamber when the cap is removably attached to the elongated stick. The RFID section is distal to the specimen collection tip, and the RFID section is capable of holding an RFID tag.

According to another embodiment of the present invention, a cryopreservation device capable of holding an RFID tag is provided. The cryopreservation device comprises a) an elongated stick comprising an RFID section, an elongated body, a frustoconical boss extending from a first end of the elongated body, and a specimen collection tip extending from the frustoconical boss, and b) a cap comprising a hollow chamber, wherein the hollow chamber has a length sufficient to accommodate the specimen collection tip and the frustoconical boss. The cap is capable of enclosing the specimen collection tip and the frustoconical boss within the hollow chamber when the cap is removably attached to the elongated stick. The RFID section is distal to the specimen collection tip, and the RFID section is capable of holding an RFID tag. The RFID section comprises a) an RFID chamber capable of holding the RFID tag, b) an RFID opening through which the RFID tag can be placed into the RFID chamber, and c) a locking mechanism abutting a circumference of the RFID opening.

According to yet another embodiment of the present invention, a cryopreservation device capable of holding an RFID tag is provided. The cryopreservation device comprises a) an elongated stick comprising an RFID section, an elongated body, a frustoconical boss extending from a first end of the elongated body, and a specimen collection tip extending from the frustoconical boss, and b) a cap comprising a hollow chamber, wherein the hollow chamber has a length sufficient to accommodate the specimen collection tip and the frustoconical boss. The cap is capable of enclosing the specimen collection tip within the hollow chamber when the cap is removably attached to the elongated stick. The RFID section is distal to the specimen collection tip, and the RFID section is capable of holding an RFID tag. The RFID section comprises a) an RFID chamber capable of holding the RFID tag, b) an RFID opening through which the RFID tag can be placed into the RFID chamber, and c) a locking mechanism abutting a circumference of the RFID opening. The elongated stick is made of one integrated piece of plastic. The RFID opening is at the end of the elongated stick. The RFID chamber comprises a cylindrical chamber outlined by plastic in a prism shape and the RFID tag is cylindrical. The prism shape has a first longitudinal slit and a second longitudinal slit, each slit starting at the RFID opening. The first longitudinal slit is 50% to 95% a length of the RFID chamber and the second longitudinal slit is 20% to 60% of the length of the RFID chamber. The first longitudinal slit is across the RFID chamber from the second longitudinal slit. The locking mechanism comprises a ledge at least partially around the inside of the RFID opening.

A process for vitrifying a biological specimen is also described, but does not form part of the present invention. The process comprises the steps of a) obtaining a cryopreservation device of any of the above embodiments, b) obtaining a liquid nitrogen resistant RFID tag, c) placing the RFID tag into the RFID chamber, reading the RFID tag and associating the RFID tag with the biological specimen, e) adding a vitrification mixture to dehydrate the biological specimen, f) collecting the dehydrated biological specimen on the elongated stick, g) enclosing the dehydrated biological specimen with a cap secured to the elongated stick, and h) placing the cryopreservation device into liquid nitrogen.

### BRIEF DESCRIPTION OF THE DRAWINGS

The present invention is illustrated and described herein with reference to the various drawings, in which like reference numbers denote like method steps and/or system components, respectively, and in which:
FIG. 1a is a side view of the cryopreservation device of Comparative Example 1;
FIG. 1b is a section view of the cryopreservation device of FIG. 1a along line A-A;
FIG. 1c is a side view of the cryopreservation device of FIG. 1a without a cap;
FIG. 1d, is a section view of the cryopreservation device of FIG. 1c along line A-A;
FIG. 2a is a side view of the cryopreservation device of the exemplary embodiment of Example 1;
FIG. 2b is a section view of the embodiment shown in FIG. 2a along line A-A;
FIG. 2c is a side view of the opposite side, (rotated 180 degrees along the longitudinal axis) of the embodiment shown in FIG. 2a;
FIG. 2d is an isometric view of the RFID section of the embodiment shown in FIG. 2a;
FIG. 3a is a side view of the exemplary embodiment of the cryopreservation device of Example 2;
FIG. 3b is a top view of the RFID section and the end of the elongated stick of embodiment shown in FIG. 3a;
FIG. 3c is an isometric view of the RFID section and the end of the elongated stick of the embodiment shown FIG. 3a;
FIG. 4a is a side view of the cryopreservation device of Comparative Example 2;
FIG. 4b is a section view of the cryopreservation device shown in FIG. 4a;
FIG. 4c is a detailed section view of the RFID section of the cryopreservation device shown in FIG. 4a;
FIG. 5a is a side view of the cryopreservation device of the exemplary embodiment of Example 3;
FIG. 5b is a section view of the embodiment shown in FIG. 5a;
FIG. 5c is a detailed section view of the RFID section of the embodiment shown in FIG. 5a;
FIG. 6a is a side view of another embodiment of the cryopreservation device with an end-loading RFID section and a plug for the locking mechanism;
FIG. 6b is a section view of the RFID section of the embodiment shown in FIG. 6a, the not-yet-loaded RFID tag and the plug detached form the RFID section;
FIG. 6c is a section view of the RFID section of the embodiment shown in FIG. 6a with an RFID tag securely contained within the RFID chamber;
FIG. 7 is a section view of another embodiment of the cryopreservation device with an end-loading RFID section;
FIG. 8a is a section view of another embodiment of the cryopreservation device with a detachable RFID section;
FIG. 8b is a section view of the detachable RFID section an elongated body of the embodiment shown in FIG. 8a in a detached state;
FIG. 8c is a section view of an alternative connection configuration of a cryopreservation device and a corresponding detachable RFID section;
FIG. 9a is a side view of another embodiment of the cryopreservation device with a top-loading RFID section;
FIG. 9b is a section view of the RFID section of the embodiment shown in FIG. 9a;
FIG. 9c is a detailed top view of the RFID section of the embodiment shown in FIG. 9a; and
FIG. 9d is different section view of the RFID section of the embodiment shown in FIG. 9c.

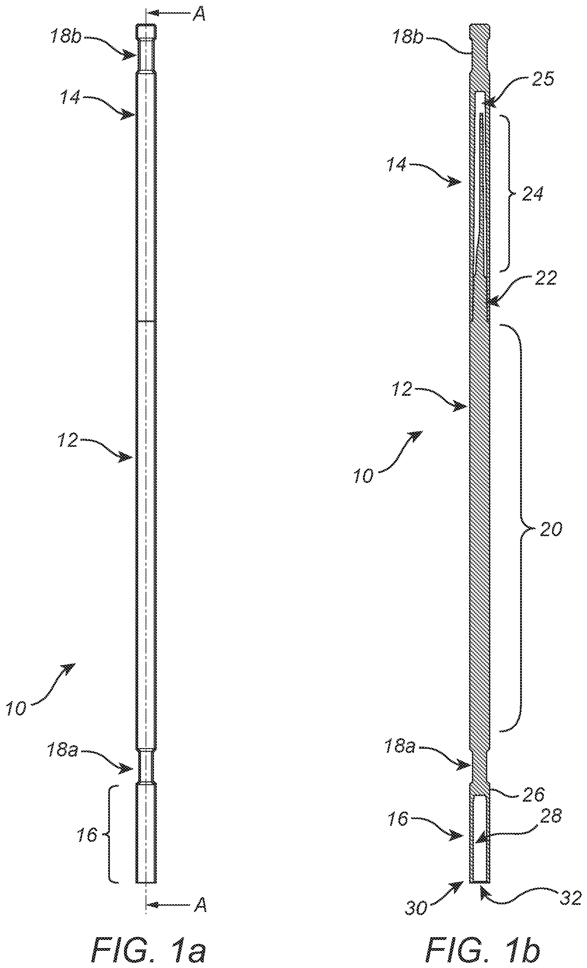

The examples and embodiments illustrated in figures 1a-d, 2a-d, 3a-c, 4a-c, 6a-c, 7, 8a-c and 9a-d are outside the scope of the claims, but useful for understanding the invention.

### DETAILED DESCRIPTION OF THE INVENTION

An embodiment of the present invention provides a cryopreservation device capable of holding a Radio Frequency Identification (RFID) tag. The cryopreservation device comprises a) an elongated stick comprising an RFID section, an elongated body, and a specimen collection tip, and b) a cap comprising a hollow chamber, wherein the hollow chamber has a length sufficient to accommodate the specimen collection tip. The cap is capable of enclosing the specimen collection tip within the hollow chamber when the cap is removably attached to the elongated stick. The RFID section is distal to the specimen collection tip and the RFID section is capable of holding an RFID tag.

The present invention may be understood more readily by reference to the following detailed description of the invention taken in connection with the accompanying drawing figures, which form a part of this disclosure. It is to be understood that this invention is not limited to the specific devices, methods, conditions, or parameters described and/or shown herein, and that the terminology used herein is for the purpose of describing particular embodiments by way of example only and is not intended to be limiting of the invention, which is defined by the appended claims.

Also, as used in the specification including the appended claims, the singular forms "a," "an," and "the" include the plural, and reference to a particular numerical value includes at least that particular value, unless the context clearly dictates otherwise.

It is to be understood that the mention of one or more method steps does not preclude the presence of additional method steps before or after the combined recited steps or intervening method steps between those steps expressly identified. Moreover, the lettering of method steps or ingredients is a conventional means for identifying discrete activities or ingredients and the recited lettering can be arranged in any sequence, unless otherwise indicated. Method steps that begin with the word "optionally" may occur, but they are not required to occur.

As used herein, the term "and/or", when used in a list of two or more items, means that any one of the listed items can be employed by itself, or any combination or two or more of the listed items can be employed. For example, if a composition is described as containing compounds A, B, "and/or" C, the composition may contain A alone; B alone; C alone; A and B in combination; A and C in combination; B and C in combination; or A, B, and C in combination.

The RFID tag is not particularly limited as long as the RFID tag is compatible with liquid nitrogen. To be compatible with liquid nitrogen, the RFID tag works as intended both at ambient conditions and when submerged in, and thus at the temperature of, liquid nitrogen. An RFID tag works as intended when it can receive an interrogation from an RFID reader and send back the RFID tag's unique identification to the reader. In some aspects, the RFID tag is one item containing an integrated circuit, an antenna, and a substrate. In some aspects, the substrate comprises silicon, and/or carbon fiber.

In a particular embodiment, the cryopreservation device further comprises an a frustoconical boss extending from a first end of the elongated body. In some aspects, the specimen collection tip extends from the frustoconical boss. In some aspects, the cap is capable of enclosing the specimen collection tip within the hollow chamber when the cap is removably attached to the elongated stick. In some aspects, the cap is capable of hermetically enclosing the specimen collection tip within the hollow chamber when the cap is removably attached to the elongated stick.

The RFID section comprises a) an RFID chamber capable of holding the RFID tag, b) an RFID opening through which the RFID tag can be placed into the RFID chamber, and c) a locking mechanism abutting a circumference of the RFID opening.

In some aspects, the elongated stick is made of one integrated piece of plastic. For example, the elongated stick is made from one integrated piece of plastic when it is injection molded in a mold that forms the RFID section, the elongated body, the specimen collection tip, and any other components of the elongated stick. In another example, the elongated stick is made from one integrated piece of plastic when it is 3D printed.

In some aspects, when the RFID tag is placed in the RFID chamber and the cryopreservation device is placed in liquid nitrogen for 5 minutes, the RFID tag remains in the RFID chamber and the cryopreservation device shows no visible cracks. In some aspects, when the RFID tag is placed in the RFID chamber and the cryopreservation device is placed in liquid nitrogen for 5 minutes, the RFID tag remains in the RFID chamber and the cryopreservation device shows no cracks with 10X magnification.

The shape of the RFID chamber corresponds to the shape of the substantially cylindrical RFID tag. In some aspects, the RFID tag has a length ranging 4 mm to 12 mm long. In some aspects, the RFID has a diameter ranging from 0.5 mm to 4 mm., 0.5 to 3.5, or 0.5 to 3 mm, or 0.5 to 2.5 mm, or 0.05 mm to 2.2 mm, or 0.5 mm to 2 mm, or 0.5 mm to 1.8 mm, or 0.5 mm to 1.4 mm, or 0.5 mm to 1.

Having extra volume, width, and/or length of the RFID chamber relative to the RFID tag may allow the RFID chamber walls to contract when immersed in liquid nitrogen while ensuring that a more rigid RFID tag does not crack the walls of the RFID chamber. In some aspects, the volume of the RFID chamber is 1.01 times the volume of the RFID tag. Other non-limiting examples include the volume of the RFID chamber being at least 1.02, or 1.03, or 1.04, or1.05, or 1.1 times the volume of the RFID tag. In some aspects, the width of the RFID chamber perpendicular to the longitudinal direction of the elongated stick is at least 1.01 or 1.02, or 1.03, or 1.04, or 1.05, or 1.1 the corresponding width of the RFID tag. In some aspects, the length of the RFID chamber along the longitudinal direction of the elongated stick is at least 1.01 or 1.02, or 1.03, or 1.04, or1.05, or 1.1 the length of the RFID tag.

In some aspects, the RFID opening is located at the end of the elongated stick. The RFID chamber comprises a substantially cylindrical chamber outlined by plastic in a prism shape. In some aspects, the prism shape is cuboidal. In some aspects, the prism shape is hexagonal. The RFID tag is substantially cylindrical, and the prism shape has a first longitudinal slit and a second longitudinal slit, each slit starting at the RFID opening. The first longitudinal slit is 50% to 95% a length of the RFID chamber and the second longitudinal slit is 20% to 60% of the length of the RFID chamber. In accordance with the invention, the first longitudinal slit is longer than the second longitudinal slit. In some aspects, the first longitudinal slit is across the RFID chamber from the second longitudinal slit.

In some aspects, the RFID section dimension in each of the x and y directions, perpendicular to the longitudinal direction, is no larger than about 5 mm. In other examples the RFID section dimension in each of the x and y directions, perpendicular to the longitudinal direction, is no larger than about 4.5 mm, or 4.0 mm, or 3.5, mm, or 3.1 mm, or 3.0 mm, or 2.8 mm, or 2.6 mm, or 2.4 mm.

In some aspects not forming part of the invention, the RFID opening is located along the longitudinal direction of the elongated stick. The RFID section comprises a locking mechanism abutting a circumference of the RFID opening. In some aspects, the locking mechanism comprises a ledge at least partially around the circumference the RFID opening. In some aspects, the length of the RFID opening is smaller than the length of the RFID tag, and the locking mechanism comprises the part of the RFID section abutting the circumference of the RFID opening and covering the RFID chamber. In some aspects, the RFID tag can be loaded into the RFID chamber at an angle to allow part of the RFID tag to enter the part of the RFID chamber that is beyond the RFID opening and then pushing the rest of the RFID tag through the RFID opening.

In some aspects not forming part of the invention, a length of the RFID opening is along a longitudinal axis of the elongated stick, wherein the RFID-opening length is shorter than a RFID-chamber length. In some aspects the locking mechanism comprises a circumferential flap bending inward toward the RFID chamber. In some aspects, the locking mechanism further comprises an obtrusion connected to the circumferential flap and protruding over RFID opening. In some aspects, the RFID chamber has at least one dimension perpendicular to the longitudinal direction that is between 1.01% to 1.1% the same dimension of the RFID tag.

A process for vitrifying a biological specimen, the process not forming part of the present invention, comprises the steps of a) obtaining a cryopreservation device of any of the above embodiments, b) obtaining a liquid nitrogen resistant RFID tag, c) placing the RFID tag into the RFID chamber, reading the RFID tag and associating the RFID tag with the biological specimen, e) adding a vitrification mixture to dehydrate the biological specimen, f) collecting the dehydrated biological specimen on the elongated stick, enclosing the dehydrated biological specimen with the cap secured to the elongated stick, and h) placing the cryopreservation device into liquid nitrogen.

In some aspects, the RFID section shows no visible cracks when placed in liquid nitrogen for 5 minutes.

### Examples

Comparative Example 1, Comparative Example 2, and Example 3 were each performed using a cryopreservation device elongated stick which was injection molded out of polystyrene. Examples 1 and 2 were performed using prototypes of the cryopreservation device elongated stick. The prototypes were 3D printed out of Digital ABS Plus from Statasys, Eden Prairie, MN. The elongated stick of each device/prototype was made of a single integrated piece of plastic. The elongated stick in each example was designed with an RFID chamber to accommodate a transponder RFID microchip tag (RFID tag) which was cylindrical with a 2 mm diameter and 12 mm length available from Shenzhen Manruta Technology Co. Ltd., Guangdong, China. The specimen collection tip of each of the elongated sticks for the Comparative Examples and the Examples was sealable with a CRYOLOCK cap (Biotech, Inc., Alpharetta, GA).

### Comparative Example 1

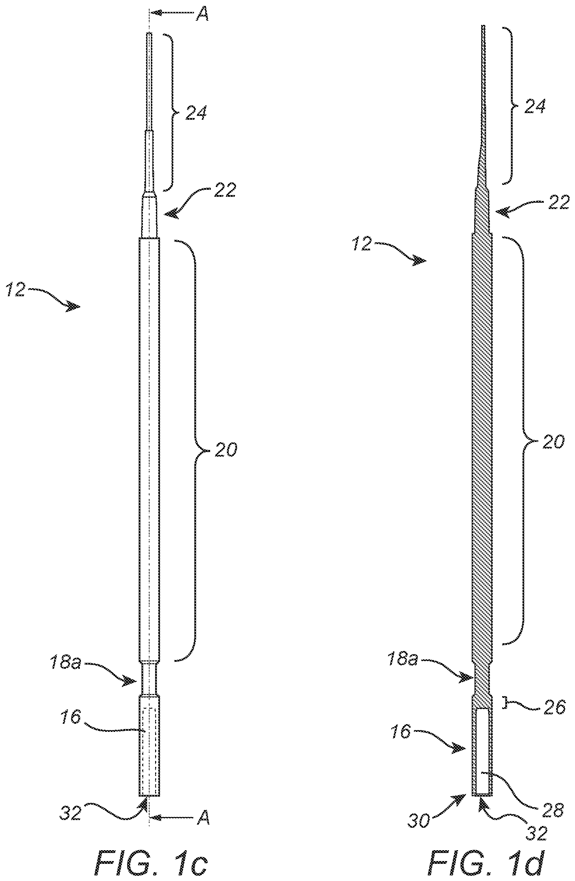

A cryopreservation device as shown in FIGs. 1a-1d was designed and injection molded. Referring to FIGs. 1a, 1b, 1c, and 1d, the cryopreservation device **10** comprised an elongated stick **12** and a cap **14.** The elongated stick comprised an RFID section **16,** a circumferential notch **18a,** an elongated body **20,** a frustoconical boss **22** extending from a first end of the elongated body **20,** and a specimen collection tip **24** extended from the narrow end of the boss **22.** The cap **14** comprised a circumferential notch **18b** and an elongated hollow chamber **25** defined along the long axis of the cap **14** and dimensioned to accommodate the specimen collection tip **24** and the frustoconical boss **22.** The circumferential notches **18a, 18b** disposed near the end of the cap **14** and the distal end of the elongated stick **12** were used to clasp the cryopreservation device **10** with forceps (not shown), making the cryopreservation device **10** easier to manipulate in the varied temperature environments. RFID section **16** comprised an RFID transition zone **26,** an RFID chamber **28,** and an RFID terminus zone **30.** The RFID terminus zone **30** was located along the longitudinal axis further from the elongated body **20** than the RFID transition zone **26,** and the RFID chamber **28** was located between the RFID terminus zone **30** and the RFID transition zone **26.** The RFID chamber **28** was sized to hold the RFID tag (not shown). As such, RFID chamber **28** was cylindrically shaped and opening **32** was circular. FIGs 1c and 1d are side and section views of the cryopreservation device **10** shown in FIG. 1 with the cap **14** removed, thus showing the entire elongated stick **12.**

An RFID tag was manually inserted into the RFID chamber **28** through opening **32** under ambient conditions. The RFID tag was securely held in RFID chamber **28,** and the chip could not be removed from the RFID chamber **28** by shaking the elongated stick **12.** The elongated stick **12** was submerged in liquid nitrogen for approximately 2 minutes. The elongated stick **12** was inspected upon removal from the liquid nitrogen. The RFID chamber 28 had several cracks visible to the naked eye.

### Example 1

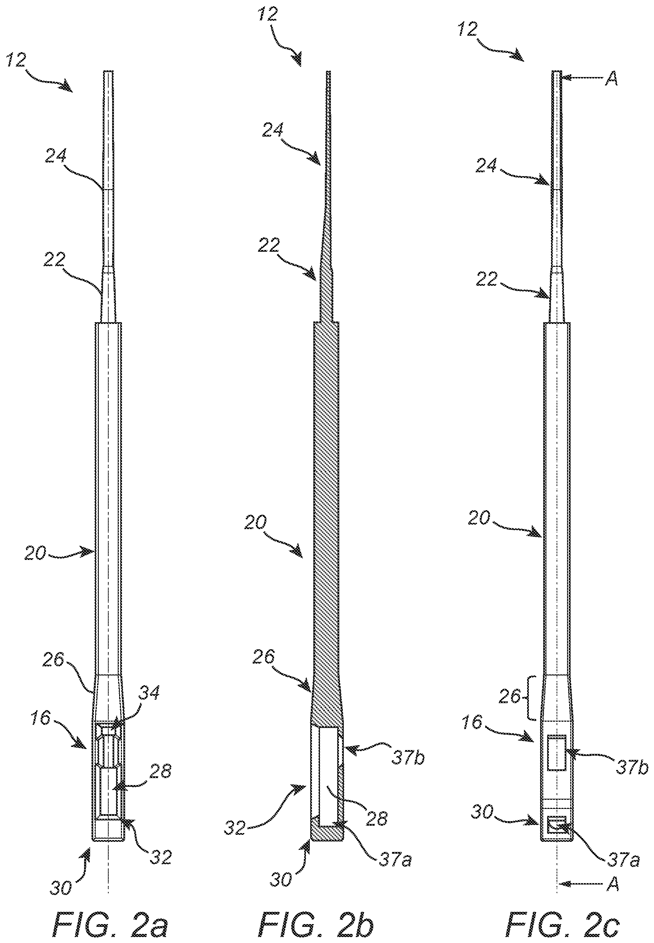

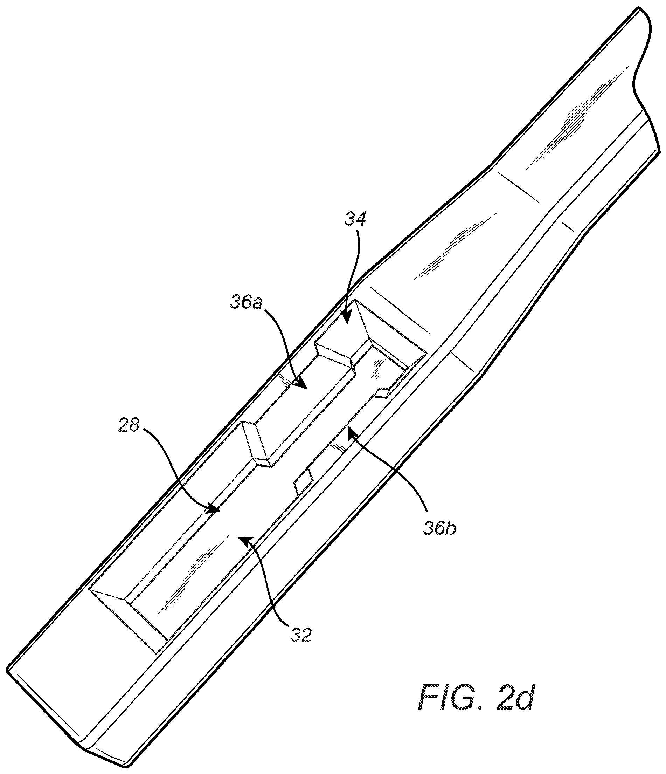

An elongated stick **12** for a cryopreservation device as shown in FIGs. 2a -2d was designed and a prototype was made via 3D printing. Referring to FIGs. 2a, 2b, 2c, and 2d the elongated stick **12** comprised an RFID section **16,** an elongated body **20,** a frustoconical boss **22** extending from a first end of the elongated body, and a specimen collection tip **24** extending from the narrow end of the frustoconical boss **22.** The RFID section **16** comprised a tapered RFID transition zone **26,** an RFID chamber **28,** and an RFID terminus zone **30.** The RFID terminus zone **30** was located along the longitudinal axis further from the elongated body **20** than the RFID transition zone **26,** and the RFID chamber **28** was located between the RFID terminus zone **30** and the tapered RFID transition zone **26.** The RFID transition zone **26** was tapered because the RFID chamber 28 had a larger cross section than the elongated body **20.** The RFID chamber **28** was sized to hold the RFID tag (not shown).

The RFID tag was manually inserted into the RFID chamber **28** through opening **32** which was sized to allow the RFID tag to pass through it and into RFID chamber **28.** Locking mechanism **34** defined opening **32.** Locking mechanism **34** shown in FIGs. 2a and 2d ensured that the RFID tag (not shown) remained inside the RFID chamber **28** throughout handling and storage of the elongated stick **12.** The RFID tag was inserted at an angle allow part of the RFID tag to enter the part of the RFID chamber **28** that was beyond the RFID opening **32** and adjacent to the RFID terminus zone **30.** As the RFID tag was then pushed into the RFID chamber **28,** flaps **36a** and **36b** bent downward and rested on top of the RFID tag once it was fully inserted into the RFID chamber **28.** As shown in FIG. 2c, openings **37a** and **37b** were spaced longitudinally along the outer edge of RFID chamber **28** and opening **32** was placed on the opposite side, perpendicularly to the longitudinal direction, of the elongated stick **12.** The RFID tag was securely held in RFID chamber **28,** and the chip could not be removed from the RFID chamber **28** by shaking the elongated stick **12.** The elongated stick **12** was submerged in liquid nitrogen. Upon being inserted into liquid nitrogen for approximately 2 minutes and then being returned to ambient conditions, the RFID section **16** showed no visible signs of cracking. The RFID section **16** was observed at 10X magnification, and still no signs of cracking were observed.

### Example 2

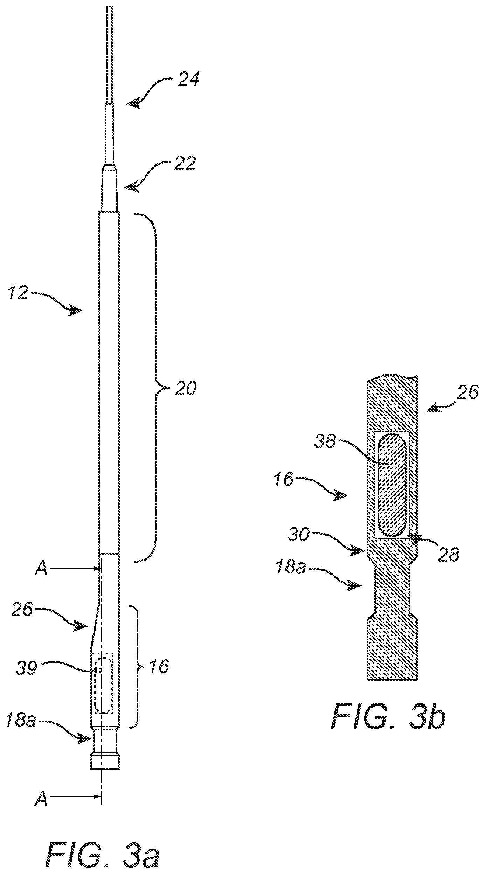

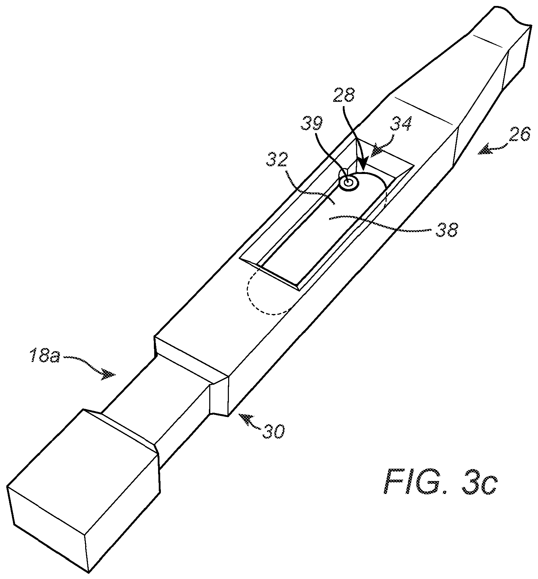

An elongated stick **12** for a cryopreservation device as shown in FIGs. 3a -3c was designed and a prototype was 3D printed. The elongated stick **12** comprised a notch **18a,** an RFID section **16,** an elongated body **20,** a frustoconical boss **22** extending from a first end of the elongated body **20,** and a specimen collection tip **24** extended from the narrow end of the frustoconical boss **22.** The RFID section **16** comprised a tapered RFID transition zone **26,** an RFID chamber **28,** and an RFID terminus zone **30.** The RFID terminus zone **30** was located along the longitudinal axis further from the elongated body **20** than the RFID transition zone **26,** and the RFID chamber **28** was located between the RFID terminus zone **30** and the RFID transition zone **26.** The RFID chamber **28** was sized to hold the RFID tag **38.** The tapered RFID transition zone **26** was designed to transition from the smaller cross section of the elongated body **20** to the larger cross section of the RFID section **16,** while allowing the resting side of elongated body **22** and the RFID section **16** to be straight for ease of handling.

An RFID tag was manually inserted into the RFID chamber **28** through opening **32** under ambient conditions. The RFID tag was inserted at an angle allow part of the RFID tag to enter the part of the RFID chamber **28** that was beyond the RFID opening **32** and adjacent to the RFID terminus zone **30.** Locking mechanism **34** included circular tag **39** which bent downward when the RFID tag **38** was loaded into the RFID chamber **28** and was above the RFID tag **38** after it was fully inserted into the RFID chamber **28.** Locking mechanism **34** kept the RFID tag **38** within the RFID chamber **28.** The RFID tag was securely held in RFID chamber **28,** and the RFID tag could not be removed from the RFID chamber **28** by shaking the elongated stick **12.** The elongated stick **12** was submerged in liquid nitrogen. Upon being inserted into liquid nitrogen for approximately 2 minutes and then being returned to ambient conditions, the RFID section **16** showed no visible signs of cracking. The RFID section **16** was observed at 10X magnification, and still no signs of cracking were observed.

### Comparative Example 2

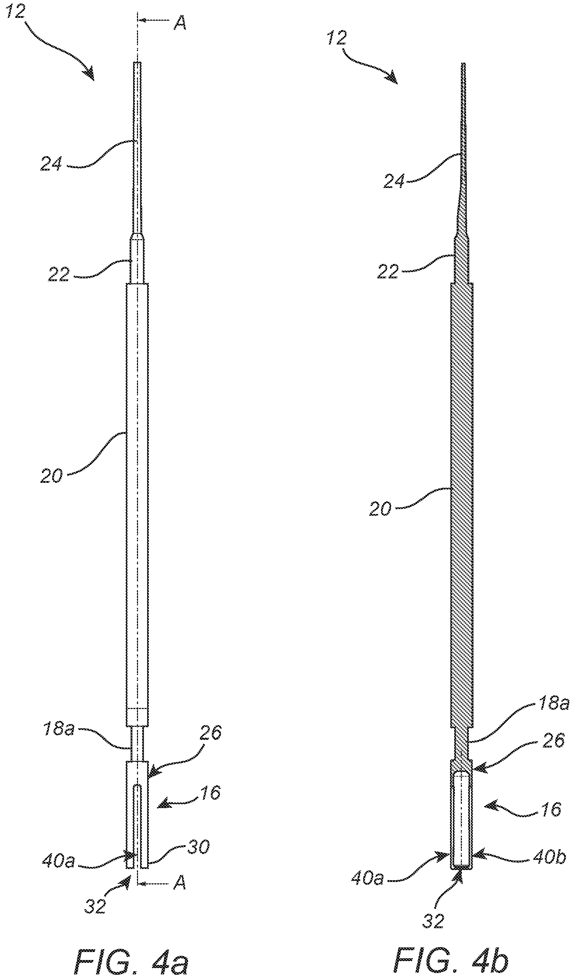

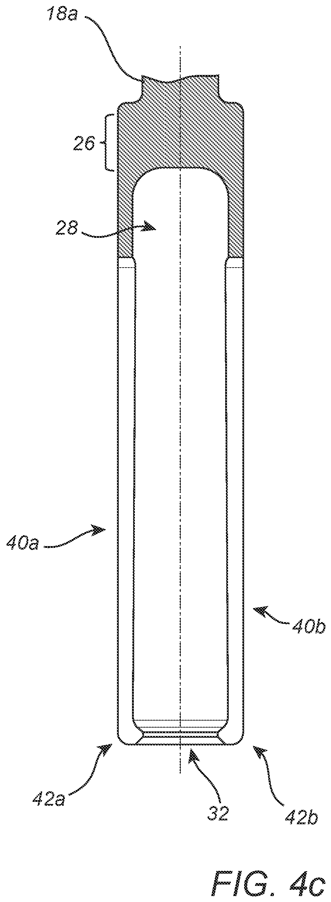

An elongated stick **12** was designed and injection molded. Referring to FIGs. 4a, 4b, and 4c, the elongated stick comprised an RFID section **16,** a circumferential notch **18a,** an elongated body **20,** a frustoconical boss **22** extending from a first end of the elongated body **20,** and a specimen collection tip **24** extended from the narrow end of the frustoconical boss **22.** The circumferential notch **18a** was located between the end of the elongated body **20** distal to the specimen collection tip **24** and the RFID section **16.** RFID section **16** comprised an RFID transition zone **26,** an RFID chamber **28,** and an RFID terminus zone **30.** The RFID terminus zone **30** was located along the longitudinal axis further from the elongated body **20** than the RFID transition zone **26,** and the RFID chamber **28** was located between the RFID terminus zone **30** and the RFID transition zone **26.** The RFID chamber **28** was sized to hold the RFID tag (not shown). As such, RFID chamber **28** was cylindrically shaped and opening **32** was circular. One of two longitudinal slits **40a** is shown in FIG. 4a, and both longitudinal slits **40a, 40b** are shown in FIGs. 4b and 4c. In this Comparative Example, the longitudinal slits were the same size, and each slit started at RFID opening **32** and removed a piece about 90% the length of the wall of the RFID chamber **28.** FIG. 4b is a section view of the elongated stick **12** shown in FIG. 4a, and FIG. 4c is a detailed sectional view of the RFID section **16** of the elongated stick **12** shown in FIG. 4a. The locking mechanism includes the "elbows" or ledge **42a, 42b** on at least part of the wall of the RFID chamber **28** nearest to RFID opening **32.**

An RFID tag was inserted into the RFID chamber **28** through opening **32** under ambient conditions. The RFID tag could not be removed from the RFID chamber **28** by shaking the elongated stick **12.** However, the RFID tag was not securely held in RFID chamber **28,** as the RFID tag could be removed from the RFID chamber **28** by simply moving the pieces of the wall of RFID chamber **28** in an outward direction. The elongated stick **12** with the RFID tag in the RFID chamber **28** was submerged in liquid nitrogen. Upon being inserted into liquid nitrogen for approximately 2 minutes and then being returned to ambient conditions, the RFID section 16 showed no visible signs of cracking. The RFID section **16** was observed at 10X magnification, and still no signs of cracking were observed. This design only addressed one of the two necessary criteria, the RFID section **16** did not crack, but the RFID tag was not securely held in place during handling of the cryopreservation device.

### Example 3

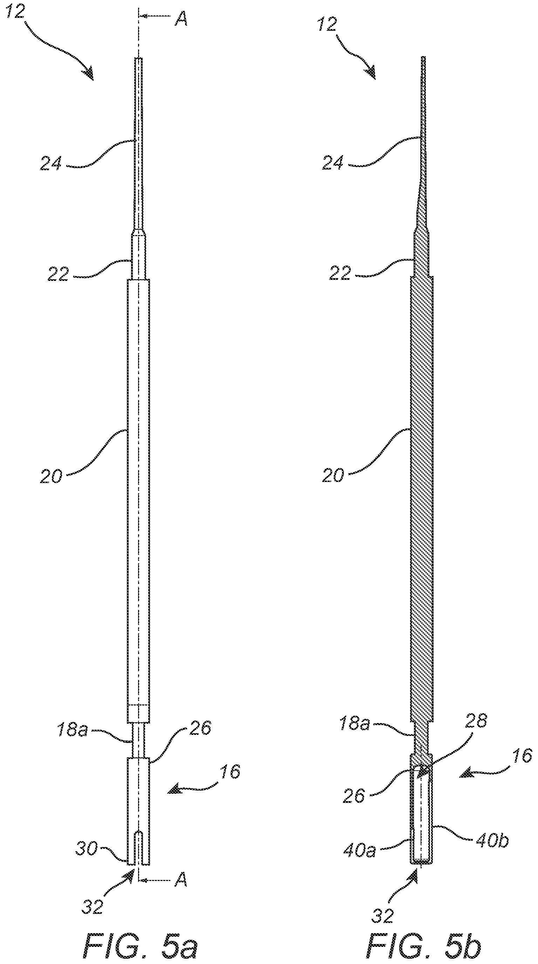

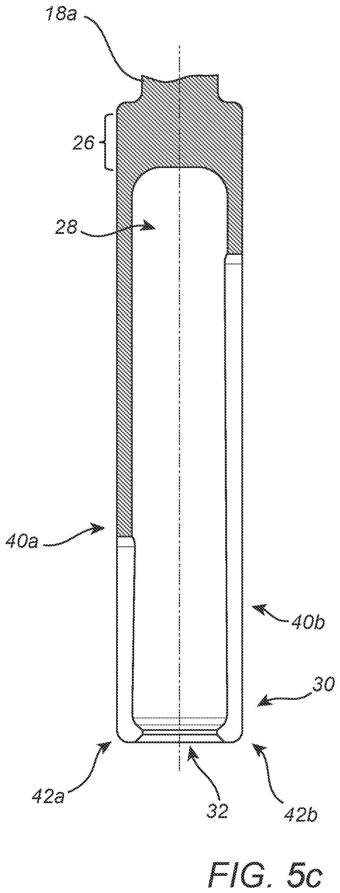

An elongated stick **12** was designed and injection molded. Referring to FIGs. 5a, 5b, and 5c, the elongated stick comprised an RFID section **16,** a circumferential notch **18a,** an elongated body **20,** a frustoconical boss **22** extending from a first end of the elongated body **20,** and a specimen collection tip **24** extended from the narrow end of the frustoconical boss **22.** The circumferential notch **18a** was located between the end of the elongated body **20** distal to the specimen collection tip **24** and the RFID section **16.** RFID section **16** comprised an RFID transition zone **26,** an RFID chamber **28,** and an RFID terminus zone **30.** The RFID terminus zone **30** was located along the longitudinal axis further from the elongated body **20** than the RFID transition zone **26,** and the RFID chamber **28** was located between the RFID terminus zone **30** and the RFID transition zone **26.** The RFID chamber **28** was sized to hold the RFID tag (not shown). As such, RFID chamber **28** was cylindrically shaped and opening **32** was circular. One of two longitudinal slits **40a** is shown in FIG. 5a, and both longitudinal slits **40a, 40b** are shown in FIGs. 5b, 5c. In this Example, the longitudinal slits are of different sizes, and longitudinal slit 40a removes a piece about 50% the length of the wall of the RFID chamber **28,** while longitudinal slit 40b removes a piece about 90% the length of the wall of the RFID chamber **28.** FIG. 5b is a section view of the elongated stick **12** shown in FIG. 5a, and FIG. 5c is a detailed sectional view of the RFID section **16** of the elongated stick **12** shown in FIG. 5a. The locking mechanism includes the "elbows" or ledge **42a, 42b** on at least part of the wall of RFID chamber **28** nearest to RFID opening **32.**

An RFID tag was inserted into the RFID chamber **28** through opening **32** under ambient conditions. The RFID tag was securely held in RFID chamber **28,** and the chip could not be removed from the RFID chamber **28** by shaking the elongated stick **12.** Unlike in Comparative Example 2, the RFID tag could not be removed from the RFID Chamber **28** by moving the walls of the RFID chamber **28** outward. The elongated stick **12** was submerged in liquid nitrogen. Upon being inserted into liquid nitrogen for approximately 2 minutes and then being returned to ambient conditions, the RFID section **16** showed no visible signs of cracking. The RFID section **16** was observed at 10X magnification, and still no signs of cracking were observed.

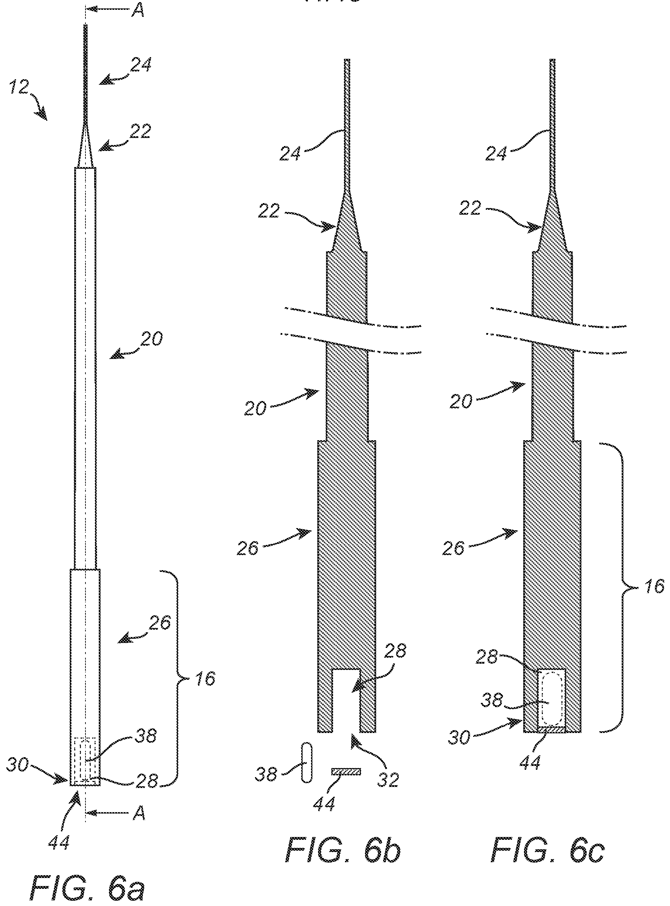

FIG. 6a illustrates another embodiment of the closed system cryopreservation device **10** with an elongated stick **12** and a cap **14** (not shown) with an end-loading RFID section **16.** The elongated stick **12** comprises an RFID section **16,** an elongated body **20,** a frustoconical boss **22** extending from a first end of the elongated body **20,** and a specimen collection tip **24** extended from the narrow end of the frustoconical boss **22.** The RFID section **16** comprises an RFID transition zone **26,** an RFID compartment **28,** and an RFID terminus zone **30.** RFID plug **44** is removably attachable to the RFID section 16. The RFID terminus zone **30** is located along the longitudinal axis further from the elongated body **20** than the RFID transition zone **26,** and the RFID chamber **28** is located between the RFID terminus zone **30** and the RFID transition zone **26.**

FIGs. 6b and 6c, are detailed sectional views of the RFID section **16** of the embodiment shown in FIG. 6a. FIG. 6b is a sectional view illustrating an empty RFID chamber **28** with the RFID tag **38** and RFID plug **44** seperate from the RFID section **16.** FIG. 6c is a sectional view illustrating RFID tag **38** within the RFID chamber **28** and secured by RFID plug **44.** In this embodiment, the RFID chamber **28** is slightly oversized relative to the RFID tag **38** to allow for thermal contraction when the cryopreservation device is submerged in liquid nitrogen. RFID plug **44** serves the same purpose as locking mechanisms **34** shown in the embodiments of FIGs. 2a - 2d and 3a - 3c. By blocking RFID opening **32,** RFID plug **44** keeps the RFID tag **38** in RFID chamber **28** throughout handling and storage of the cryopreservation device.

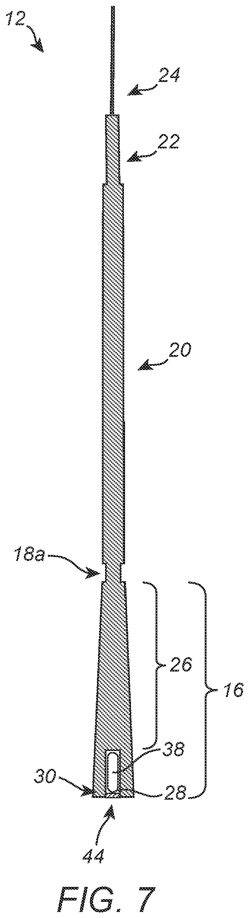

FIG. 7 illustrates another embodiment of the cryopreservation device **10** with an elongated stick **12** and a cap **14** (not shown) with an alternative end-loading RFID section **16.** The elongated stick **12** comprises an RFID section **16,** a circumferential notch **18a,** an elongated body **20,** frustoconical boss **22** extending from a first end of the elongated body **20,** and a specimen collection tip **24** extended from the narrow end of the boss **22.** The circumferential notch **18a** is located between the end of the elongated body **20** distal to the specimen collection tip **24** and the RFID section **16.** The RFID section **16** comprises an RFID transition zone **26,** an RFID chamber **28,** and an RFID terminus zone **30** containing an RFID plug **44.** The RFID terminus zone **30** is located along the longitudinal axis further from the elongated body **20** than the RFID transition zone **26,** and the RFID chamber **28** is located between the RFID terminus zone **30** and the RFID transition zone **26.** In FIG. 7, the entirety of the RFID section **16** is tapered, giving it a larger cross section the further removed from the elongated body **20.**

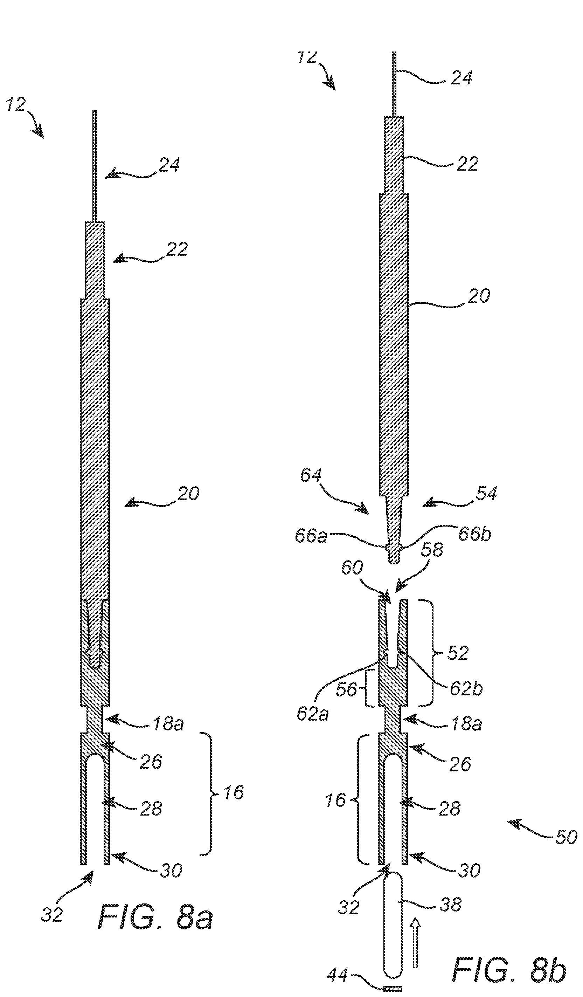

FIGs. 8a and 8b illustrate another embodiment of the cryopreservation device **10** with an elongated stick **12** and a cap **14** (not shown). The elongated stick **12** comprises a removably attachable component **50,** an elongated body **20,** frustoconical boss **22** extending from a first end of the elongated body, and a specimen collection tip **24** extended from the narrow end of the frustoconical boss **22.** The removably attachable component **50** comprises RFID section **16,** notch **18a,** and RFID connection element **52.** The elongated body **20** comprises a compatible body connection element **54.** RFID connection element **52** comprises transition zone **56** and RFID locking mechanism **58.** Transition zone **56** is distal to elongated body **20** and RFID locking mechanism **58** is between transition zone **56** and elongated body **20** when removably attachable component **50** is attached to the elongated body **20.** RFID locking mechanism **58** comprises a hollow chamber **60** with notches **62a** and **62b** toward the end of hollow chamber **60** nearest transition zone **56.** The compatible body connection element **54** is located on elongated body **20** distal from the specimen collection tip **24** and comprises solid object **64** and extensions **66a** and **66b.** Extensions **66a** and **66b** are located toward the end of compatible body connection element **54** distal to elongated body **20.** Solid object **64** is sized to fit in chamber **60** and extensions **66a** and **66b** are sized to fit into notches **62a** and **62b,** when removably attachable component **50** is attached to the elongated body **20.**

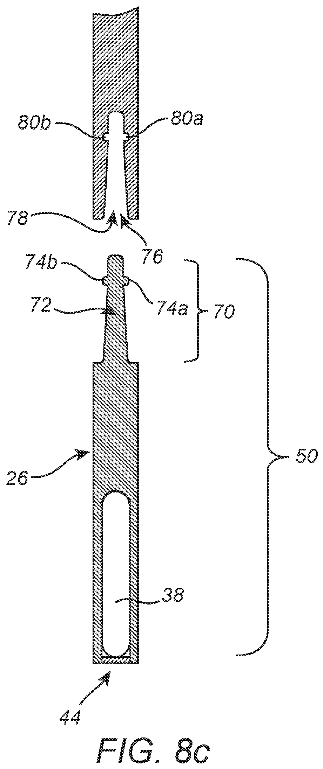

FIG. 8c illustrates an alternative locking mechanism for the RFID connection element **52** of the removably attachable component **50** and the compatible body connection element **54** shown in FIG. 8b. The removably attachable RFID connection element **50** comprises RFID section **16** and RFID connection element **70.** RFID connection element **70** comprises solid object **72** with extensions **74a** and **74b.** RFID transition zone **26** is distal to elongated body **20** and solid object **72** is between RFID transition zone **26** and the elongated body **20** when removably attachable component **50** is attached to the elongated body **20.** Extensions **74a** and **74b** are located toward the end of solid object **72** distal from RFID transition zone **26.** The compatible body locking mechanism **76** comprises a hollow chamber **78** with notches **80a** and **80b.** Compatible body locking mechanism **76** is sized to receive solid object **72** and notches **80a** and **80b** are sized to receive extensions **74a** and **74b,** when removably attachable component **50** is attached to the elongated body **20.**

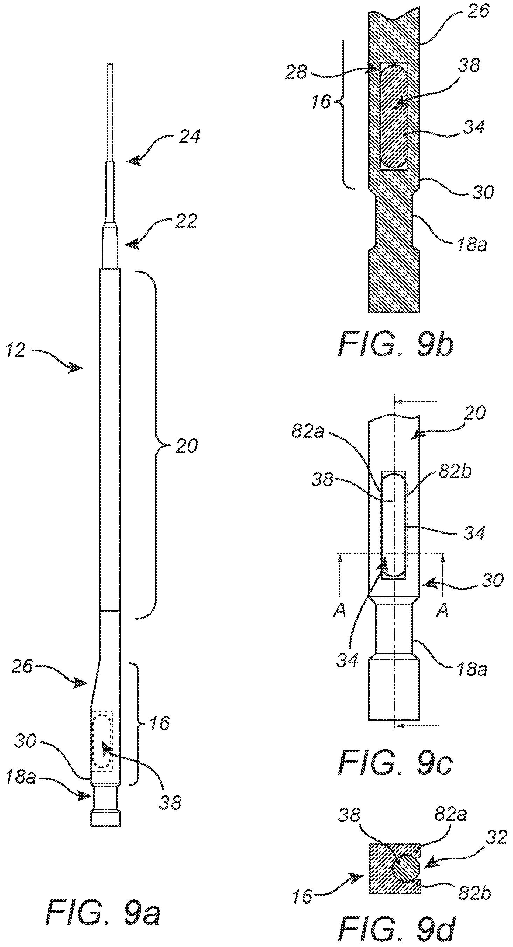

FIG. 9a illustrates another embodiment of the cryopreservation device **10** with an elongated stick **12** and a cap **14** (not shown) with another embodiment of top-loading RFID section **16.** The elongated stick **12** comprises a notch **18a,** an RFID section **16,** an elongated body **20,** a frustoconical boss **22** extending from a first end of the elongated body **20,** and a specimen collection tip **24** extended from the narrow end of the frustoconical boss **22.** The RFID section **16** comprises an RFID transition zone **26,** an RFID chamber **28,** and an RFID terminus zone **30.**

FIGs. 9b, 9c, and 9d are detailed views of the RFID section **16** of the embodiment shown in FIG. 9a. FIG. 9b is a section view illustrating an RFID tag **38** in the RFID chamber **28.** The edges of RFID tag **38** are not seen because the locking mechanism **34** runs the length of the RFID chamber **28,** and the locking mechanism sits on top of the sides of the RFID tag **38** once it is loaded into the RFID chamber **28.** FIG. 9c is a top view illustrating the locking mechanism **34** which defines opening **32** at the top of the RFID chamber **28.** Locking mechanism parts **82a** and **82b** extend over the RFID tag **38** when the RFID tag **38** is fully inserted into the RFID chamber **28.** Locking mechanism parts **82a** and **82b** are capable of keeping the RFID tag **38** within the RFID chamber **28.**

## Claims

1. A cryopreservation device (10), the cryopreservation device (10) comprising:
a) an elongated stick (12) comprising an RFID section (16), an elongated body (20), and a specimen collection tip (24), and
b) a cap (14) comprising a hollow chamber (25), wherein the hollow chamber (25) has a length sufficient to accommodate the specimen collection tip (24),
wherein the cap (14) is capable of enclosing the specimen collection tip (24) within the hollow chamber (24) when the cap (14) is removably attached to the elongated stick (12),
wherein the RFID section (16) is distal to the specimen collection tip (24), and
wherein the RFID section (16) comprises:
a) an RFID chamber (28) capable of holding an RFID tag,
b) an RFID opening (32) through which the RFID tag can be placed into the RFID chamber (28), and
c) a locking mechanism abutting a circumference of the RFID opening (32), and
wherein the RFID opening (32) is at an end of the elongated stick (12), **characterized in that** the RFID chamber (28) comprises a substantially cylindrical chamber outlined by plastic in a prism shape, wherein the RFID tag is substantially cylindrical, and wherein the prism shape has a first longitudinal slit (40a) and a second longitudinal slit (40b), each slit starting at the RFID opening (32), wherein the first longitudinal slit (40a) is 50% to 95% a length of the RFID chamber (28) and the second longitudinal slit (40b) is 20% to 60% of the length of the RFID chamber (28), and wherein the first longitudinal (40a) slit is longer than the second longitudinal slit (40b).

2. The cryopreservation device (10) of claim 1, wherein the elongated stick (12) is made of one integrated piece of plastic.

3. The cryopreservation device (10) of claim 1, wherein the RFID tag has a length ranging from 4 mm to 12 mm long and a diameter ranging from 0.5 mm to 4 mm.

4. The cryopreservation device (10) of claim 1, wherein the first longitudinal slit (40a) is across the RFID chamber (28) from the second longitudinal slit (40b).

5. The cryopreservation device (10) of claim 1, wherein the locking mechanism comprises a ledge (42a, 42b) at least partially around the circumference of the RFID opening (32).

6. The cryopreservation device (10) of claim 1, wherein the RFID section (16) dimension in each of the x and y directions, perpendicular to the longitudinal direction, is no larger than about 3 mm.

7. The cryopreservation device (10) of claim 1, wherein the RFID section (16) dimension in each of the x and y directions, perpendicular to the longitudinal direction, is no larger than about 2.4 mm.

8. The cryopreservation device (10) of claim 1, wherein the elongated stick (12) further comprises a frustoconical boss (22) extending from a first end of the elongated body (20), the specimen collection tip (24) extends from the frustoconical boss (22), the hollow chamber (25) of the cap (25) has a length sufficient to accommodate the specimen collection tip (24) and the frustoconical boss (22), and the cap (14) is capable of enclosing the specimen collection tip (24) and the frustoconical boss (22) within the hollow chamber (25) when the cap (14) is removably attached to the elongated stick (12).

9. The cryopreservation device (10) of claim 8, wherein the elongated stick (12) is made of one integrated piece of plastic, and wherein the RFID tag has a length ranging from 6 mm to 12 mm long and a diameter ranging from 0.5 mm to 4 mm.

10. The cryopreservation device (10) of claim 8, wherein the locking mechanism comprises a ledge (42a, 42b) at least partially around the RFID opening (32).

11. The cryopreservation device (10) of claim 10, wherein the first longitudinal slit (40a) is about 90% a length of the RFID chamber (28) and the second longitudinal slit (40b) about 50% of the length of the RFID chamber (28), and wherein the first longitudinal slit (40a) is across the RFID chamber (28) from the second longitudinal slit (40b).

12. The cryopreservation device (10) of claim 8, wherein the RFID section (16) dimension in each of the x and y directions, perpendicular to the longitudinal direction, is no larger than about 2.4 mm.

13. The cryopreservation device (10) of claim 8, wherein the elongated stick (12) is made of one integrated piece of plastic.

14. The cryopreservation device (10) of claim 8,
wherein the first longitudinal slit (40a) is across the RFID chamber (28) from the second longitudinal slit (40b), and
wherein the locking mechanism comprises a ledge (42a, 52b)at least partially around the circumference of the RFID opening (32).

## Patentansprüche

1. Kryokonservierungsvorrichtung (10), die aufweist:
a) einen länglichen Stab (12), der einen RFID-Abschnitt (16), einen länglichen Körper (20) und eine Probenaufnahmespitze (24) aufweist, und
b) eine Kappe (14), die eine Hohlkammer (25) aufweist, wobei die Hohlkammer (25) eine zur Aufnahme der Probenaufnahmespitze (24) ausreichende Länge aufweist,
wobei die Kappe (14) dazu geeignet ist, die Probenaufnahmespitze (24) innerhalb der Hohlkammer (25) zu umschließen, wenn die Kappe (14) lösbar an dem länglichen Stab (12) angebracht ist,
wobei der RFID-Abschnitt (16) distal zur Probenaufnahmespitze (24) angeordnet ist und
wobei der RFID-Abschnitt (16) aufweist:
a) eine RFID-Kammer (28), die ein RFID-Tag aufnehmen kann,
b) eine RFID-Öffnung (32), durch die das RFID-Tag in die RFID-Kammer (28) eingebracht werden kann, und
c) einen Verriegelungsmechanismus, der an dem Umfang der RFID-Öffnung (32) anliegt, und
wobei sich die RFID-Öffnung (32) an einem Ende des länglichen Stabs (12) befindet, **dadurch gekennzeichnet, dass** die RFID-Kammer (28) eine im Wesentlichen zylindrische Kammer umfasst, die von prismatisch geformtem Kunststoff umgrenzt ist, wobei das RFID-Tag im Wesentlichen zylindrisch ist und wobei die Prismenform mit einem ersten Längsschlitz (40a) und einem zweiten Längsschlitz (40b) versehen ist, wobei jeder Schlitz an der RFID-Öffnung (32) beginnt, wobei der erste Längsschlitz (40a) eine Länge von 50 % bis 95 % der Länge der RFID-Kammer (28) aufweist und der zweite Längsschlitz (40b) eine Länge von 20 % bis 60 % der Länge der RFID-Kammer (28) aufweist und wobei der erste Längsschlitz (40a) länger als der zweite Längsschlitz (40b) ist.

2. Kryokonservierungsvorrichtung (10) nach Anspruch 1, wobei der längliche Stab (12) aus einem einzigen einstückigen Kunststoffteil gefertigt ist.

3. Kryokonservierungsvorrichtung (10) nach Anspruch 1, wobei das RFID-Tag eine Länge im Bereich von 4 mm bis 12 mm und einen Durchmesser im Bereich von 0,5 mm bis 4 mm aufweist.

4. Kryokonservierungsvorrichtung (10) nach Anspruch 1, wobei der erste Längsschlitz (40a) auf einer dem zweiten Längsschlitz (40b) gegenüberliegenden Seite der RFID-Kammer (28) angeordnet ist.

5. Kryokonservierungsvorrichtung (10) nach Anspruch 1, wobei der Verriegelungsmechanismus einen Randvorsprung (42a, 42b) umfasst, der sich zumindest teilweise entlang des Umfangs der RFID-Öffnung (32) erstreckt.

6. Kryokonservierungsvorrichtung (10) nach Anspruch 1, wobei die Abmessung des RFID-Abschnitts (16) sowohl in der x-Richtung als auch in der y-Richtung, jeweils senkrecht zu der Längsrichtung, nicht größer als etwa 3 mm ist.

7. Kryokonservierungsvorrichtung (10) nach Anspruch 1, wobei die Abmessung des RFID-Abschnitts (16) sowohl in der x-Richtung als auch in der y-Richtung, jeweils senkrecht zu der Längsrichtung, nicht größer als etwa 2,4 mm ist.

8. Kryokonservierungsvorrichtung (10) nach Anspruch 1, wobei der längliche Stab (12) ferner einen kegelstumpfförmigen Ansatz (22) aufweist, der sich von einem ersten Ende des länglichen Körpers (20) erstreckt, wobei sich die Probenaufnahmespitze (24) von dem kegelstumpfförmigen Ansatz (22) erstreckt, wobei die Hohlkammer (25) der Kappe (14) eine zur Aufnahme der Probenaufnahmespitze (24) und des kegelstumpfförmigen Ansatzes (22) ausreichende Länge aufweist und wobei die Kappe (14) dazu geeignet ist, die Probenaufnahmespitze (24) und den kegelstumpfförmigen Ansatz (22) innerhalb der Hohlkammer (25) zu umschließen, wenn die Kappe (14) lösbar an dem länglichen Stab (12) angebracht ist.

9. Kryokonservierungsvorrichtung (10) nach Anspruch 8, wobei der längliche Stab (12) aus einem einzigen einstückigen Kunststoffteil gefertigt ist und wobei das RFID-Tag eine Länge im Bereich von 6 mm bis 12 mm und einen Durchmesser im Bereich von 0,5 mm bis 4 mm aufweist.

10. Kryokonservierungsvorrichtung (10) nach Anspruch 8, wobei der Verriegelungsmechanismus einen Randvorsprung (42a, 42b) umfasst, der sich zumindest teilweise um die RFID-Öffnung (32) herum erstreckt.

11. Kryokonservierungsvorrichtung (10) nach Anspruch 10, wobei der erste Längsschlitz (40a) eine Länge von etwa 90 % der Länge der RFID-Kammer (28) aufweist und der zweite Längsschlitz (40b) eine Länge von etwa 50 % der Länge der RFID-Kammer (28) aufweist und wobei der erste Längsschlitz (40a) auf einer dem zweiten Längsschlitz (40b) gegenüberliegenden Seite der RFID-Kammer (28) angeordnet ist.

12. Kryokonservierungsvorrichtung (10) nach Anspruch 8, wobei die Abmessung des RFID-Abschnitts (16) sowohl in der x-Richtung als auch in der y-Richtung, jeweils senkrecht zur Längsrichtung, nicht größer als etwa 2,4 mm ist.

13. Kryokonservierungsvorrichtung (10) nach Anspruch 8, wobei der längliche Stab (12) aus einem einzigen einstückigen Kunststoffteil gefertigt ist.

14. Kryokonservierungsvorrichtung (10) nach Anspruch 8,
wobei der erste Längsschlitz (40a) auf einer dem zweiten Längsschlitz (40b) gegenüberliegenden Seite der RFID-Kammer (28) angeordnet ist und
wobei der Verriegelungsmechanismus einen Randvorsprung (42a, 42b) umfasst, der sich zumindest teilweise entlang des Umfangs der RFID-Öffnung (32) erstreckt.

## Revendications

1. Dispositif de cryoconservation (10), ledit dispositif de cryoconservation (10) comprenant :
a) une tige allongée (12) comprenant une section de RFID (16), un corps allongé (20) et un embout de prélèvement d'échantillon (24), et
b) un capuchon (14) comprenant une chambre creuse (25), dans lequel la chambre creuse (25) a une longueur suffisante pour accueillir l'embout de prélèvement d'échantillon (24),
dans lequel le capuchon (14) est capable d'enfermer l'embout de prélèvement d'échantillon (24) au sein de la chambre creuse (25), lorsque le capuchon (14) est fixé de manière amovible à la tige allongée (12),
dans lequel ladite section de RFID (16) est distale par rapport à l'embout de prélèvement d'échantillon (24), et
dans lequel ladite section de RFID (16) comprend :
a) une chambre de RFID (28) capable de contenir une étiquette RFID,
b) une ouverture de RFID (32) par laquelle l'étiquette RFID peut être placée dans la chambre de RFID (28), et
c) un mécanisme de verrouillage venant buter contre la circonférence de l'ouverture de RFID (32), et
dans lequel l'ouverture de RFID (32) est située à une extrémité de la tige allongée (12), **caractérisé en ce que** la chambre de RFID (28) comprend une chambre sensiblement cylindrique délimitée par du plastique en forme de prisme, dans lequel ladite étiquette RFID est sensiblement cylindrique, et dans lequel la forme de prisme comporte une première fente longitudinale (40a) et une seconde fente longitudinale (40b), chaque fente commençant à l'ouverture de RFID (32), dans lequel la première fente longitudinale (40a) a une longueur qui est 50 % à 95 % de la longueur de la chambre de RFID (28) et la seconde fente longitudinale (40b) a une longueur qui est 20 % à 60 % de la longueur de la chambre de RFID (28), et dans lequel la première fente longitudinale (40a) est plus longue que la seconde fente longitudinale (40b).

2. Dispositif de cryoconservation (10) selon la revendication 1, dans lequel la tige allongée (12) est constituée d'une seule pièce de plastique monobloc.

3. Dispositif de cryoconservation (10) selon la revendication 1, dans lequel l'étiquette RFID présente une longueur comprise entre 4 mm et 12 mm et un diamètre compris entre 0,5 mm et 4 mm.

4. Dispositif de cryoconservation (10) selon la revendication 1, dans lequel la première fente longitudinale (40a) est située de l'autre côté de la chambre de RFID (28) par rapport à la seconde fente longitudinale (40b).

5. Dispositif de cryoconservation (10) selon la revendication 1, dans lequel le mécanisme de verrouillage comprend un rebord (42a, 42b) s'étendant au moins partiellement autour de la circonférence de l'ouverture de RFID (32).

6. Dispositif de cryoconservation (10) selon la revendication 1, dans lequel la dimension de la section de RFID (16) dans chacune des directions x et y, perpendiculaires à la direction longitudinale, n'est pas supérieure à environ 3 mm.

7. Dispositif de cryoconservation (10) selon la revendication 1, dans lequel la dimension de la section de RFID (16) dans chacune des directions x et y, perpendiculaires à la direction longitudinale, n'est pas supérieure à environ 2,4 mm.

8. Dispositif de cryoconservation (10) selon la revendication 1, dans lequel la tige allongée (12) comprend en outre un bossage tronconique (22) s'étendant à partir d'une première extrémité du corps allongé (20), l'embout de prélèvement d'échantillon (24) s'étend à partir du bossage tronconique (22), la chambre creuse (25) du capuchon (14) a une longueur suffisante pour accueillir l'embout de prélèvement d'échantillon (24) et le bossage tronconique (22), et le capuchon (14) est capable d'enfermer l'embout de prélèvement d'échantillon (24) et le bossage tronconique (22) au sein de la chambre creuse (25), lorsque le capuchon (14) est fixé de manière amovible à la tige allongée (12).

9. Dispositif de cryoconservation (10) selon la revendication 8, dans lequel la tige allongée (12) est constituée d'une seule pièce de plastique monobloc, et dans lequel l'étiquette RFID présente une longueur comprise entre 6 mm et 12 mm et un diamètre compris entre 0,5 mm et 4 mm.

10. Dispositif de cryoconservation (10) selon la revendication 8, dans lequel le mécanisme de verrouillage comprend un rebord (42a, 42b) s'étendant au moins partiellement autour de l'ouverture de RFID (32).

11. Dispositif de cryoconservation (10) selon la revendication 10, dans lequel la première fente longitudinale (40a) a une longueur qui est environ 90 % de la longueur de la chambre de RFID (28) et la seconde fente longitudinale (40b) a une longueur qui est environ 50 % de la longueur de la chambre de RFID (28), et dans lequel la première fente longitudinale (40a) est située de l'autre côté de la chambre de RFID (28) par rapport à la seconde fente longitudinale (40b).

12. Dispositif de cryoconservation (10) selon la revendication 8, dans lequel la dimension de la section de RFID (16) dans chacune des directions x et y, perpendiculaires à la direction longitudinale, n'est pas supérieure à environ 2,4 mm.

13. Dispositif de cryoconservation (10) selon la revendication 8, dans lequel la tige allongée (12) est constituée d'une seule pièce de plastique monobloc.

14. Dispositif de cryoconservation (10) selon la revendication 8,
dans lequel la première fente longitudinale (40a) est située de l'autre côté de la chambre de RFID (28) par rapport à la seconde fente longitudinale (40b), et
dans lequel le mécanisme de verrouillage comprend un rebord (42a, 42b) s'étendant au moins partiellement autour de la circonférence de l'ouverture de RFID (32).
